# EUROPEAN PATENT APPLICATION

(11) **EP 1 439 163 A1**
(43) Date of publication of application: **21.07.2004**
(21) Application number: 01974665.0
(22) Date of filing: 03.10.2001
(51) Int. Cl.: C07C 69/743, C07C 67/08, A01N 53/00

(54) **PROPARGYLBENZYL ALCOHOL ESTER DERIVATIVE, PROCESS FOR PRODUCING THE SAME, AND INSECTICIDE/INSECTIFUGE CONTAINING THE SAME**

(71) Applicant: DAINIHON JOCHUGIKU CO., LTD., Osaka-shi Osaka 550-0001 (JP)
(72) Inventor: INOUE, Masafumi, c/o DAINIHON JOCHUGIKU CO. LTD, Toyonaka-shi, Osaka 561-0827 (JP); NAKAYAMA, Koji, c/o DAINIHON JOCHUGIKU CO. LTD, Toyonaka-shi, Osaka 561-0827 (JP)
(74) Representative: HOFFMANN - EITLE
(86) International application number: PCT/JP2001/008700
(87) International publication number: WO 2003/031388

(57) **Abstract**

A propargyl benzyl alcohol ester derivative represented by formula (I): 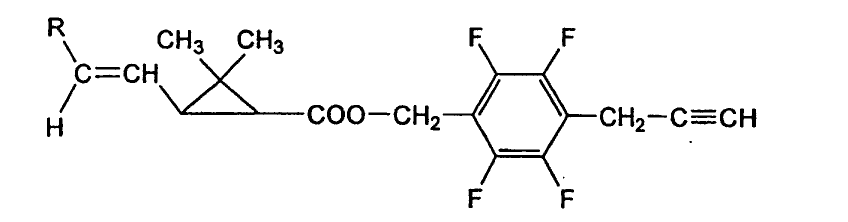 wherein R represents a hydrogen atom, a methyl group, an ethyl group, a methoxycarbonyl group or an ethoxycarbonyl group is provided. Also, a method for producing the derivative and an insecticidal or moth proofing agent containing the derivative as an active ingredient are provided. The derivative has fast-acting property and lethal effect simultaneously, and is highly safe and excellent in volatility.

## Description

### Technical Field

The present invention relates to novel and useful propargyl benzyl alcohol ester derivatives, to a method for producing the same, and to an insecticidal or moth proofing agent containing the derivative as an active ingredient.

### Background Art

Various types of benzyl alcohol ester-based insecticides have been known. Typical examples thereof include phenothrin, which is chrysanthemic acid ester of 3-phenoxybenzyl alcohol, and so forth. These benzyl alcohol ester-based pyrethroids have various properties. For example, they have high insecticidal activity and chemical stability. They can be synthesized relatively simply and easily. They are low in toxicity to warm blooded animals. However, they have poor volatility and are difficult to apply to fields where high volatility is required. Accordingly, benzyl alcohol ester-based pyrethroids which fluorine atoms are introduced on the benzyl moiety thereof is substituted with have been developed as disclosed in, for example, Japanese Patent Laid-open No. Sho 61-207361 and Japanese Patent Publication No. Hei 7-29989. However, they are not fully satisfactory and development of more useful compounds has been increasingly demanded.

Therefore, an object of the present invention is to solve the above problems of the conventional insecticidal or insect proofing ingredients and provide effective compounds having high safety and excellent volatility as well as fast-acting property and lethal effect simultaneously and useful in many respects.

Another object of the present invention is to provide a method for producing such compounds.

Still another object of the present invention is to provide an insecticidal or moth proofing agent containing such compounds as an active ingredient.

### Disclosure of Invention

With a view to achieving the above-mentioned objects, the present inventors have made extensive research and as a result they have found out novel propargyl benzyl alcohol ester derivatives represented by formula (I): 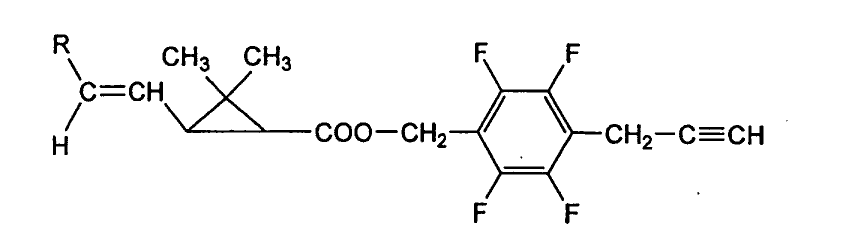 wherein R represents a hydrogen atom, a methyl group, an ethyl group, a methoxycarbonyl group or an ethoxycarbonyl group, and confirmed that they can be put into practical use. The present invention is based on the discovery.

That is, the invention as set forth in claim 1 relates to a novel propargyl benzyl alcohol ester derivative represented by formula (I) above. The esters represented by formula (I) above include optical isomers based on the stereostructure of cyclopropane ring or geometric isomers. The present invention includes such optical isomers and geometric isomers individually or any optional mixtures thereof.

Novel propargyl benzyl alcohol ester derivatives represented by formula (I) include the following compounds:
(1) Compound 1
   4-Propargyl-2,3,5,6-tetrafluorobenzyl 2 ,2-dimethyl-3-(1-propenyl) cyclopropanecarboxylate;
(2) Compound 2
   4-Propargyl-2,3,5,6-tetrafluorobenzyl 2,2-dimethyl-3-vinylcyclopropanecarboxylate;
(3) Compound 3
   4-Propargyl-2,3,5,6-tetrafluorobenzyl 2,2-dimethyl-3-(1-butenyl) cyclopropanecarboxylate;
(4) Compound 4
   4-Propargyl-2,3,5,6-tetrafluorobenzyl 2,2-dimethyl-3-(3-methoxy-3-oxo-1-propenyl) cyclopropanecarboxylate; and
(5) Compound 5

4-Propargyl-2,3,5,6-tetrafluorobenzyl 2,2-dimethyl-3-(3-ethoxy-3-oxo-1-propenyl) cyclopropanecarboxylate.

4-Propargyl-2,3,5,6-tetrafluorobenzyl alcohol is already known. For example, Japanese Patent Laid-open No. Sho 61-207361 discloses an ester that corresponds to the combination of it with 2,2-dimethyl-3-(2,2-dichlorovinyl)cyclopropanecarboxylic acid. Under the circumstances, the present inventors paid attention to the carboxylic acid that constitutes the formula (I) and tested various combinations thereof with the above-mentioned alcohol component. As a result, they have found out that the compounds represented by formula (I) are more useful than the pyrethroids currently put into practice.

The invention as set forth in claims 2 and 3 selects Compounds 1 and 4, respectively, which are particularly useful compounds among the compounds represented by formula (I) above.

The invention as set forth in claim 4 relates to a method for producing a novel propargyl benzyl alcohol ester derivative represented by formula (I) as set forth in claim 1, comprising reacting a carboxylic acid represented by formula (IV): 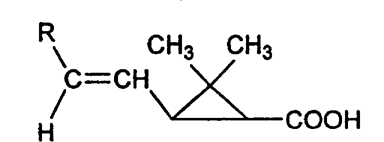 wherein R is as defined above, or its reactive derivative with an alcohol represented by formula (V): 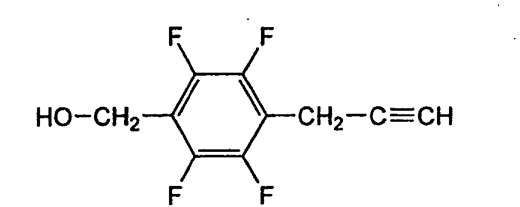 or its reactive derivative.

The reactive derivatives of the carboxylic acid include, for example, acid halides, acid anhydrides, carboxylic acid lower alkyl esters, alkali metal salts or salts with organic tertiary bases of carboxylic acid. On the other hand, reactive derivatives of the alcohol include, for example, chlorides, bromides, p-toluenesulfonic acid esters and so forth of the alcohol. The reaction may be carried out in a suitable solvent, optionally in the presence of an organic or inorganic base or acid as a deacidificating agent or a catalyst with optional heating.

In a preferred embodiment of the production method of the present invention, the carboxylic acid and the alcohol undergo esterification in the presence of dicyclohexylcarbodiimide and 4-dimethylamino pyridine.

In the case where an ester having a preferred stereostructure is to be produced, a method of isolating the resultant ester using an optically active resolving agent may be used. Usually, however, a method of synthesizing the carboxylic acid or alcohol with a preferred stereostructure and then supplying it to esterification is more practical.

The invention as set forth in claim 5 relates to an insecticidal or moth proofing agent comprising as an active ingredient a propargyl benzyl alcohol ester derivative represented by formula (I): 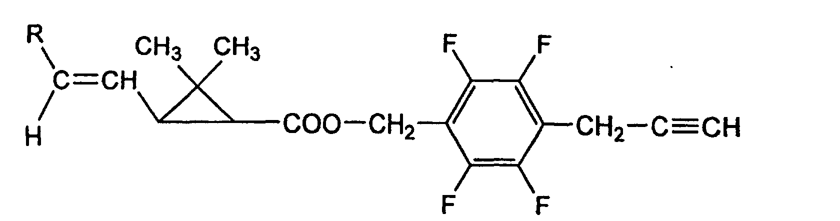 wherein R represents a hydrogen atom, a methyl group, an ethyl group, a methoxycarbonyl group or an ethoxycarbonyl group.

The invention as set forth in claims 6 and 7 selects Compounds 1 and 4, respectively, as an active ingredient in the insecticidal or moth proofing agent comprising the novel propargyl benzyl alcohol ester derivative as set forth in claim 5.

### Best Mode for carrying out the Invention

The compounds represented by formula (I) are novel compounds that are solid or liquid at ambient temperature. They are generally readily soluble in organic solvents.

When using the compounds of the present invention in practice, they may be used alone without adding other components. However, generally they are used together with a carrier that is blended thereto in order to improve their usability as an insecticidal or moth proofing agent.

Insecticidal or insect proofing preparations for spraying include emulsifiable concentrate, oil solution, dust, wettable powder, aerosol preparation and so on. They can be prepared by a method that is well known to one skilled in the art with optionally blending emulsifiers, dispersants, solvents, auxiliaries such as stabilizers, solid carriers, liquid carriers, propellants and so forth to the above-described compounds.

The insecticidal or insect proofing preparations of the present invention may be mixed with a suitable substrate such as wood powder so that they can be used as insecticidal or moth proofing agent for fumigation such as a mosquito coil. Furthermore, the above-described compound may be dissolved in a suitable organic solvent and the solution is impregnated in a mat and heated by a suitable heater to vaporize them. Alternatively, the compound may be dissolved in a suitable solvent and the solution is absorbed through a suitable liquid absorbing wick and heated by a suitable heater to vaporize it. Thus, when used in a so-called electric mosquito repellent vaporizer, the compounds and insecticidal or moth proofing agent of the present invention exhibit excellent effects similarly to those in the case of a mosquito coil. Since the compounds of the present invention are excellent in volatility as compared with the conventional pyrethroids, the temperature of the heater in an electric mosquito repellent vaporizer can be set at a temperature of 70 to 150°C, which temperature is lower than in the case of using the conventional preparations.

The carriers that can be used in preparing the compounds of the present invention in the form of dust typically include various mineral powders such as silicic acid, kaolin, and talc, various plant material powders such as wood powder and wheat flour, and so on. Organic foaming agents such as azodicarbonamide, combustion heat generators such as potassium chlorate, and so forth may be blended to prepare fumigants. In this case, similarly excellent effects can be obtained.

Furthermore, the compounds of the present invention usually are carried on or in a substrate such as a mat or sheet by impregnating, coating or the like and can be vaporized at ambient temperature preferably using a fan or the like. Thus, they find a wide application as insecticidal or moth proofing agent. The mat- or sheet-like substrate includes pulp-made mat, paper, woven fabric, nonwoven fabric, molded articles of plastics such as polyethylene, polypropylene, polyvinyl chloride, polyester, and ethylene-vinyl acetate copolymer, porous glass material, and so forth.

Also sublimating carriers made of, for example, adamantane, cyclododecane, triisopropyl/trioxane and so forth may be used in order to control the volatility of the compounds of the present invention. Alternatively, the compounds of the present invention may be prepared in the form of gel using gelling agents such as polyvinyl alcohol, alginic acid, and carrageenan.

The insecticidal or moth proofing agent of the present invention may contain synergists such as N-octylbicycloheptenecarboxyimide (trade name: MGK-264), a mixture of N-octylbicycloheptenedicarboxyimide and arylsulfonic acid salt (trade name: MGK-5026), Synepirine 500, octachlorodipropyl ether, and piperonyl butoxide.

Furthermore, other insecticidal or insect proofing components, for example, organophosphorus agents such as fenitrothion, DDVP, and diazinon, carbamate agents such as NAC, MTMC, metoxadiazone, and propoxur, conventional pyrethroids-based insecticides such as pyrethrin, allethrin, prallethrin, furamethrin, phthalthrin, phenothrin, permethrin, and empenthrin, organosilicon-based compounds such as silafluofen, antibacterial agents, antifungal agents, repellents, perfumes, deodorants and so forth may be mixed in order to obtain multi-purpose compositions with excellent effect. Accordingly, manpower can be saved and in addition synergistic effects among the chemicals can be well expected.

As for applications of the insecticidal or moth proofing agent of the present invention, it exhibits high insecticidal or insect proofing effects on sanitary insect pests such as flies, mosquitoes, cockroaches, and household dust mites; clothing insect pests such as clothes moth, webbing clothes moth, and carpet beetle; and stored grain insect pests such as rice weevil, as well as various insect pests such as aphids, leafhoppers, stink bugs, centipedes, and chironomid.

Hereinafter, synthesis examples of the compounds used in the present invention will be illustrated.

### Synthesis Example 1

### IR 3300 cm⁻¹ (-CH₂-C≡CH), 1730 cm⁻¹ (-COO-)

2.3 g of 2,2-dimethyl-3-(1-propenyl)cyclopropanecarboxylic acid chloride was dissolved in 15 ml of dry benzene. To this was added a solution of 2.1 g of 4-propargyl-2,3,5,6-teterafluorobenzyl alcohol in 10 ml of dry benzene. Further, 3 ml of dry pyridine as a condensing agent was added to the mixture to precipitate crystals of pyridine hydrochloride. The vessel was stoppered and left standing at room temperature overnight and then the crystals of pyridine hydrochloride were filtered. The benzene solution was dried over Glauber's salt, and the benzene was distilled off under reduced pressure to obtain 3.5 g of 4-propargyl-2,3,5,6-tetrafluorobenzyl 2,2-dimethyl-3-(1-propenyl)cyclopropanecarboxylate.

### Synthesis Example 2

### IR 3300 cm⁻¹ (-CH₂-C≡CH), 1730 cm⁻¹ (-COO-)

To a solution of 2.2 g of 1R,cis-2,2-dimethyl-3-[1-(ΔZ)-3-methoxy-3-oxo-1-propenyl]cyclopropanecarboxylic acid and 2.1 g of 4-propargyl-2,3,5,6-tetrafluorobenzyl alcohol in 30 ml of dichloromethane was added a solution of 2.1 g of dicyclohexylcarbodiimide and 0.2 g of 4-dimethylaminopyridine in 20 ml of dichloromethane under ice cooling. The resulting mixture was stirred at room temperature for 12 hours and then refluxed for 2 hours to complete the reaction. Dicyclohexylurea precipitated after cooling was filtered off. The filtrate was concentrated to obtain oily substance, which was passed through a column packed with 100 g of silica gel to obtain 3.7 g of 4-propargyl-2,3,5,6-tetrafluorobenzyl 1R,cis-2,2-dimethyl-3-[1-(ΔZ)-3-methoxy-3-oxo-1-propenyl]cyclopropanecarboxylate.

Next, the superiority of insecticidal or moth proofing agent containing novel propargyl benzyl alcohol ester derivatives of the present invention will be illustrated hereinbelow by examples and efficacy test results.

### Example 1

White kerosene was added to 0.2 part of compound of the invention to make 100 parts to obtain a 0.2% oil solution.

### Example 2

0.4 Part of compound of the invention and 1.0 part of MGK-5026 were added to 98.6 parts of a substrate for mosquito coil such as pyrethrum extract powder, wood powder and starch and the mixture was uniformly blended. The resulting mixture was processed by a conventional method to obtain a mosquito coil.

### Example 3

Kerosene was added to 0.3 g of compound of the invention to make 100 ml. The resulting solution was filled into a vessel for aerosol. After attaching a valve portion to the vessel, 200 ml of a propellant (a mixed gas composed of liquefied petroleum gas and dimethyl ether) was filled under pressure into the vessel through the valve portion. Thus an aerosol was obtained.

### Example 4

0.3 Part of compound of the invention and 99.7 parts of clay were well pulverized and mixed to obtain a 0.3% dust.

### Example 5

3.0 Parts of compound of the invention, 3.0 parts of metoxadiazone and 94.0 parts of azodicarbonamide were well mixed. Then 20 g of the mixture was filled in a plastic film bag. This was placed in a heat resistant vessel and an igniter was attached to obtain a fumigant.

### Efficacy Test Example 1 Insecticidal Test using an Oil Solution

A 0.2°to white kerosene solution of the compound of the invention (A), a mixture of a 0.2% white kerosene solution of the compound of the invention and a 0.8% white kerosene solution of Synepirine 500 (B), 0.2% white kerosene solutions of phthalthrin and phenothrin, Control Compound 1 [4-propargyl-2,3,5,6-tetrafluorobenzyl 2,2-dimethyl-3-(2,2-dichlorovinyl)cyclopropanecarboxylate] (the compound disclosed in Japanese Patent Laid-open No. Sho 61-207361), and Control Compound 2 [4-propargyl-2,3,5,6-tetrafluorobenzyl 2,2-dimethyl-3-(2-fluoro-3-methoxy-3-oxo-1-propenyl)cyclopropanecarboxylate] (the compound disclosed in Japanese Patent Publication No. Hei 7-29989), respectively, were evaluated on the ratio of knocked-down individuals for house fly. The relative effective concentrations of the test chemicals were calculated. Furthermore, mortality after 24 hours was obtained. The results obtained are shown in Table 1. The numbers in the brackets "()" indicate mortality after 24 hours.

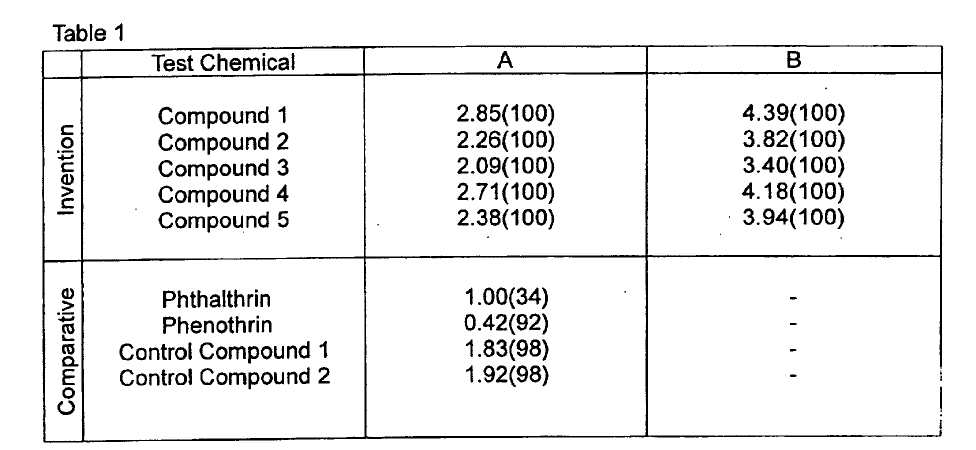

As a result, the compounds of the present invention revealed to have knockdown effect superior to phthalthrin known to be a knockdown agent and excellent mortality as compared with phenothrin known to be a killing agent. In addition, the compounds of the present invention were found to be more useful insecticidal or insect proofing component than Control Compounds 1 and 2. Furthermore, it was further evidenced that blending of Synepirine 500, a synergist to conventional pyrethroids can increase the insecticidal or insect proofing effect of the compounds of the present invention.

### Efficacy Test Example 2 Insecticidal Test Using a Mosquito Coil

In a 70 cm³ glass chamber were released about 50 adult mosquitoes *Culex pipiens pallens.* A battery-driven small fan (blade size = 13 cm in diameter) was placed in the chamber and energized. Then, 0.1 g of mosquito coil containing Compound 1, 2 and 3 of the invention obtained in Example 2, ignited at the both ends thereof was placed in the chamber. In this case, 80% or more of the mosquitoes were knocked down within 30 minutes. On day next, 80% or more of the mosquitoes were killed.

### Efficacy Test Example 3 Insecticidal Test Using a Fumigant

A pack of fumigant containing Compound 4 and 5 of the invention prepared in accordance with Example 5 was heated to about 250°C by a heater in a 6-mat room. As a result the effective component was diffused through smoke holes formed in the plastic film bag into the room. The fumigant revealed to be effective to control household dust mites such as *Dermatophagoides farinae* and *Tyrophagus putrescentiae* as well as cockroach, flea, and bedbug.

### Efficacy Test Example 4 Insecticidal Test Using Aerosol

In a 60-cm³ chamber were released about 30 female adult house flies and an aerosol containing Compound 1, 2 and 4 prepared in Example 3 was sprayed for 1 second through a hole formed in the side wall of the chamber. As a result, 100% of the houseflies were knocked down within 2 minutes. Also, the mortality was determined to be 100%. Thus, it was evidenced that the compounds of the present invention have both knockdown effect and lethal effect simultaneously.

### Effect Test Example 5 Insecticidal Test Using an Ambient Temperature Vaporizing Agent

200 mg of Compound 1 and 2 of the invention was impregnated in 3 g of granular foamed cellulose beads (Trade Name: VISCOPEARL manufactured by Rengo Co., Ltd.) having an average outer diameter of 6 mm to obtain a chemical-impregnated preparation. This was charged in a cylindrical cartridge having an outer diameter of 5 cm and a height of 3 cm. The cartridge was formed of 3 mm wide opening slits at intervals of 3 mm in the full height of the side wall thereof.

The cartridge was attached to a vaporizer equipped with a fan and the vaporizer was placed in the center of a 6-mat room. The fan was rotated at 1500 rpm. As a result, the cartridge was effective for controlling mosquitoes for 30 days.

### Industrial Applicability

The novel propargyl benzyl alcohol ester derivatives of the present invention represented by formula (I) above are useful compounds. Insecticidal or moth proofing agent containing such a compound as active ingredient has both fast-acting effect and lethal effect simultaneously as well as high safety and excellent volatility, so that it is more useful than insecticidal or moth proofing agents containing conventional pyrethroid compounds.

## Claims

1. A propargyl benzyl alcohol ester derivative represented by formula (I): 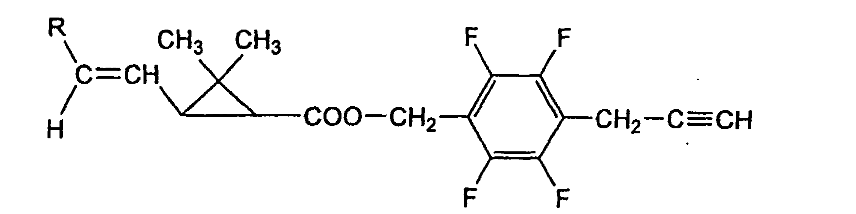 wherein R represents a hydrogen atom, a methyl group, an ethyl group, a methoxycarbonyl group or an ethoxycarbonyl group.

2. A propargyl benzyl alcohol ester derivative according to claim 1, represented by formula (II):

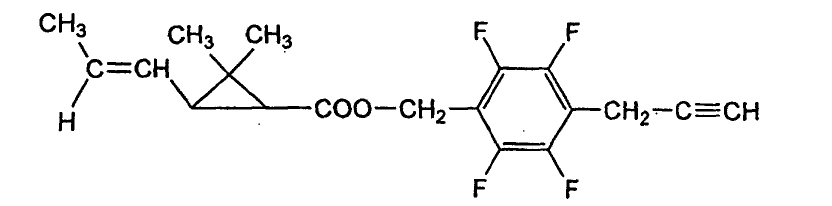

3. A propargyl benzyl alcohol ester derivative according to claim 1, represented by formula (III):

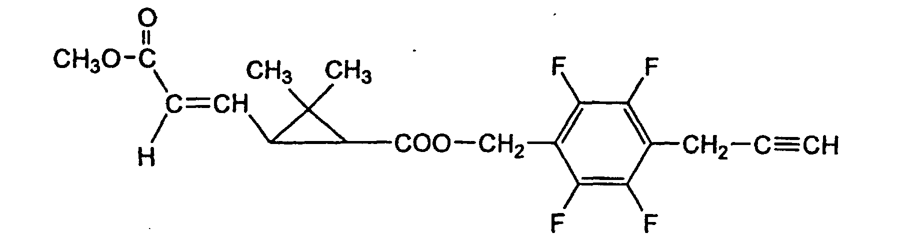

4. A method for producing a propargyl benzyl alcohol ester derivative represented by formula (I): 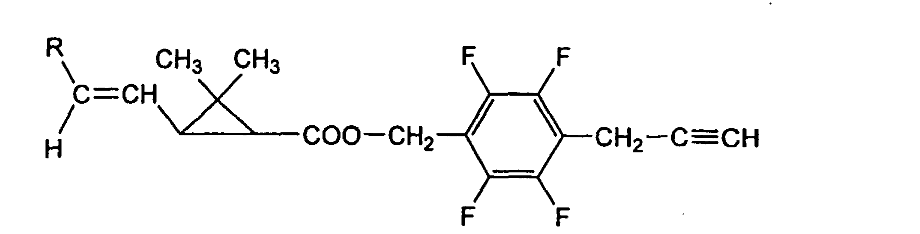 wherein R represents a hydrogen atom, a methyl group, an ethyl group, a methoxycarbonyl group or an ethoxycarbonyl group, comprising the step of reacting a carboxylic acid represented by formula (IV): 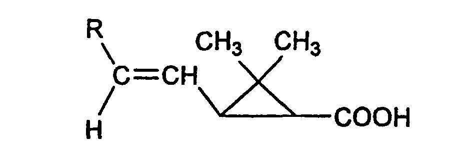 wherein R is as defined above, or its reactive derivative with an alcohol represented by formula (V): 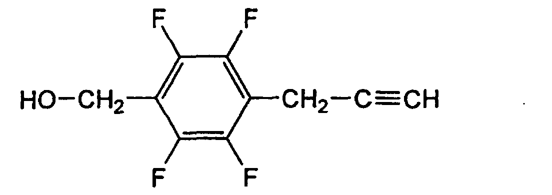 or its reactive derivative.

5. An insecticidal or moth proofing agent comprising as an active ingredient a propargyl benzyl alcohol ester derivative represented by formula (I): 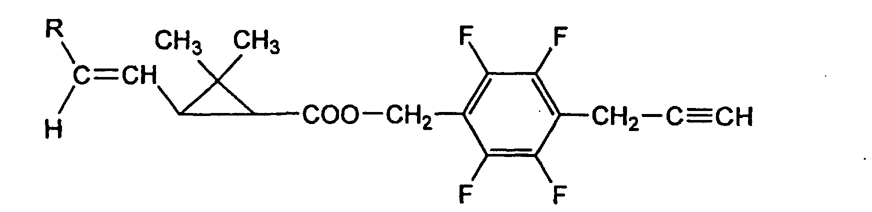 wherein R represents a hydrogen atom, a methyl group, an ethyl group, a methoxycarbonyl group or an ethoxycarbonyl group.

6. An insecticidal or moth proofing agent according to claim 5, wherein said propargyl benzyl alcohol ester derivative is represented by formula (II):

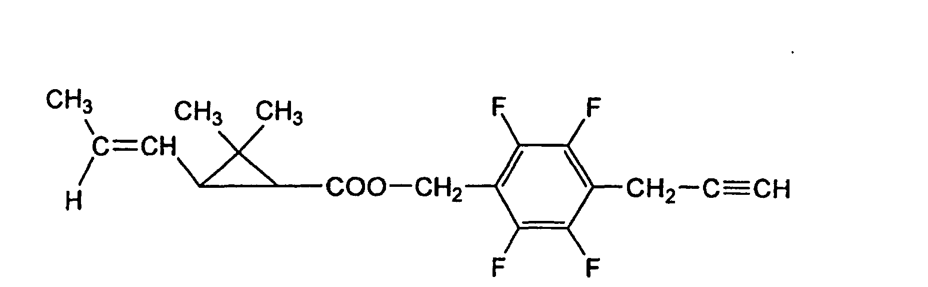

7. An insecticidal or moth proofing agent according to claim 5, wherein said propargyl benzyl alcohol ester derivative is represented by formula (III):
